# EUROPEAN PATENT SPECIFICATION

(11) **EP 3 669 292 B1**
(45) Date of publication and mention of the grant of the patent: **23.09.2026**
(21) Application number: 18855376.2
(22) Date of filing: 01.08.2018
(51) Int. Cl.: G06F 21/32, G06F 21/60, G06F 21/62, A61B 5/1172, H04L 9/32, G06F 21/64

(54) **COLLECTING APPARATUS AND COLLECTING METHOD**
SAMMELVORRICHTUNG UND SAMMELVERFAHREN
APPAREIL ET PROCÉDÉ DE COLLECTE

(30) Priority: 18.09.2017 US 201715707431
(43) Date of publication of application: 24.06.2020
(73) Proprietor: Schneider, David, Lyle, Hong Kong (CN)
(72) Inventor: Schneider, David, Lyle, Hong Kong (CN)
(74) Representative: Isern Patentes y Marcas S.L.
(86) International application number: PCT/US2018/044823
(87) International publication number: WO 2019/055147

(56) References cited:
- US-A1- 2002 124 537
- US-A1- 2005 229 007
- US-A1- 2012 293 642
- US-A1- 2015 036 065
- US-A1- 2015 067 348
- US-A1- 2015 235 226

## Description

### FIELD OF THE INVENTION

The present invention relates to a collecting apparatus and a collecting method which reads, records, and encodes patient biometric data.

### BACKGROUND OF THE INVENTION

Medical device technology and the systems providing health-care service to public populations have progressed exponentially during recent years following computing revolution in the early 1970's and personal computing revolutions since the 1980's.

This is well-known history and the public health benefits deriving from these technical and informational advancements are very important and significant for citizens of many countries. However, along with this progress, there are unanticipated challenges created by the complexity and inter-connectedness of medical and health-care industry systems.

One recent risk has been the proliferation of 'hacker' activity with purpose of causing damage and disruption to others based upon personal, political, nation-state and economic objectives. For medical industry, this means patients have valid concerns about the privacy, accuracy and disclosure of their very sensitive health-related and person-related information.

Current industry trends address these problems with ubiquitous security solutions focused on applications, databases, firewalls, and activity alarm systems. One part of the solution is, for example, proprietary encrypting hard drives which are manufactured and installed in servers and workstations to protect against unauthorized disclosure. And, in short, there is a public problem that requires multiple solutions to protect privacy of individuals and patients. Privacy expectations are extremely sensitive in medical health-care. US20120293642A1 relates to biometric enrollment kiosks for capturing, for example, face, iris, fingerprint, signature, and document data. US20150235226A1 relates to a method of witnessed fingerprint payment, and devices embodying the method.

### SUMMARY OF THE INVENTION

The object of the present invention is to
overcome above deficiencies by providing a collecting apparatus and a collecting method which improves patient privacy when using a biometric signature such as fingerprints, face scans and related characteristics when recording patient information into a computing system.

The invention is set out in the appended claims.

With this collecting apparatus and collecting method described above, patient privacy can be protected while recording some highly private and personal data about an individual. This is a challenging solution considering the high-level government and insurance industry goals, in a systematic way, demand collecting biometric information (i.e. fingerprints, photographs, other data based upon personal characteristics of an individual).

The present invention has been briefly described above. Details of the present invention will be further described in the embodiments below with the accompanied drawings.

### BRIEF DESCRIPTION OF THE DRAWINGS

Fig.1 is a perspective view schematically showing an overall construction of a collecting apparatus of a preferred embodiment of the present invention.
Fig.2 is a schematic diagram showing hardware mechanisms in the collecting apparatus of Fig.1.
Fig.3 is a flowchart showing a collecting method of a preferred embodiment of the present invention.

In the figures:
1: casing module; 2: exhaust vent screen; 3: intake vent filter; 4: display screen; 5: metal heat-sink casing; 6: two camera modules; 7: fingerprint bar reader; 8: thumbprint reader;

### DETAILED DESCRIPTION OF THE INVENTION

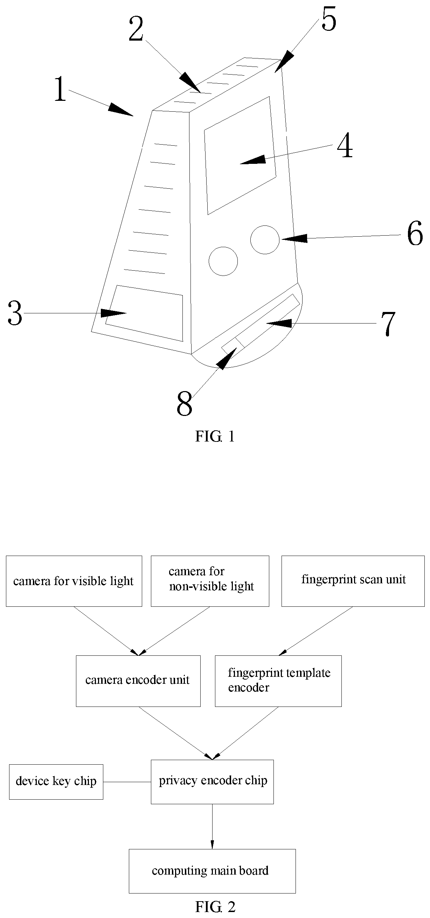

Referring to Fig. 1, a collecting apparatus is described. Fig.1 is a perspective view schematically showing an overall construction of a collecting apparatus of a preferred embodiment of the present invention.

The collecting apparatus comprises: a casing module 1 with filtered vents; an exhaust vent screen 2; an intake vent filter 3 which is necessary to remove particulate matter and improve reliability of apparatus in imperfect and hot field conditions; an display screen 4 which displays instructions provided to the patient; a metal heat-sink casing 5 for camera heat; two camera modules 6, of which normal visible light for one camera and thermal, infrared or non-visible light spectrum for the other; a fingerprint bar reader 7 for multiple-finger scanning; and a thumbprint reader 8 used for single-finger scanning.

the collecting apparatus has a trapezoidal shape in its side view and a substantially rectangular shape in its front view. An exhaust vent screen 2 is formed in the top part and side part of the collecting apparatus, through which air flows out; while an intake vent filter 3 is formed at the bottom part of the collecting apparatus, through which the air flows in.

The display screen 4 is provided in the front face of the apparatus and has a rectangular shape. A camera module is provided below the display screen 4, in the camera module, there are two different cameras aligned in the height direction, one of the two cameras is for visible light and the other is for non-visible light.

The casing 5 is made of material that is easy to dissipate the heat generated by the camera module, preferably, made of metals.

A fingerprint collecting module is provided in the bottom part of the collecting apparatus. The fingerprint collecting module includes two fingerprint readers, a fingerprint reader 7 and a fingerprint reader 8 that are provided adjacent to each other. Among the two fingerprint readers, one fingerprint reader which is elongated shape is used for multiple-finger scanning, while the other fingerprint reader is used for single-finger scanning, for example, thumb scanning.

Inside the casing module, there is provided a purging module configured to purge all temporary data acquired by the photo collecting module and the fingerprint collecting module.

Hardware mechanisms in the collecting apparatus of Fig.1 will be described with reference to Fig.2. Fig.2 is a diagram showing hardware mechanisms in the collecting apparatus of Fig.1.

As shown, this diagram indicates the relationship and order of hardware mechanisms in the collecting apparatus in Fig.1. The process begins at the top with an acquisition hardware (two camera modules 6), where raw biometric data is input. This unprotected data is encoded by hardware before delivery to temporary storage on an encryption chip mechanism. The collecting apparatus utilizes a private encryption key which is known only to the apparatus. And the hardware encryption mechanism is marked privacy encoder chip to illustrate this final step.

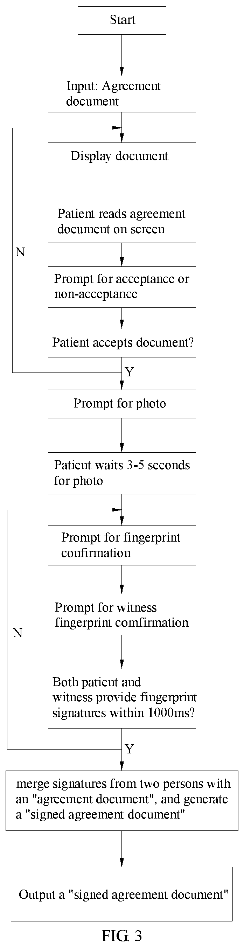

A collecting method of a preferred embodiment of the present invention is described with reference to Fig.3. Fig.3 is a flowchart showing a collecting method of a preferred embodiment of the present invention. This flowchart demonstrates how a private biometric signature can be used to confirm a real-time medical transaction. For medical fraud prevention, these steps demonstrate how a patient can review a document and then certify with a witness, using a real-time apparatus, with hardware encryption.

In the first step, an agreement document is input and displayed.

Then, the collecting apparatus of the present invention prompts for acceptance or non-acceptance. In this step, if the patient acknowledges and accepts the document, the apparatus prompts for photo, and the patient waits 3-5 seconds for photo; and, if the patient does not accept the document, the process returns back to the first step.

After taking the photo successfully, the process goes on to the next step, where biometric fingerprints are required to confirm the identities of the patient and the witness, wherein, the fingerprints from two persons are recorded on two physical reader devices.

Next, the collecting apparatus time-stamps each biometric fingerprints and electronically determines that the fingerprints are recorded within a certain time period, for example, 10 seconds; then computing an electronic decision about the physical proximity of one person and one witness based upon the first fingerprint reader time-stamp and the second fingerprint reader time-stamp. If it is determined that the fingerprints are recorded within 10 seconds, the apparatus merges biometric signatures, from two persons, with an "agreement document", generating a "signed agreement document"; and if it is determined that the fingerprint are not recorded within 10 seconds, the process returns to the fingerprint scan step.

Then the process goes on to the last step where the "signed agreement document" is output to a computing main board, and the process is finished by now.

## Claims

1. A collecting apparatus for recording a medical transaction declaration, comprising:
an input module configured for inputting patient information;
a computing main board;
a photo collecting module including a display unit (4) configured to display countdown timer instructions for taking photos, a camera unit (6) configured to take photos, an encoder unit configured to combine and hash the photos into a combined data set and forward the combined data set to a temporary storage device on a privacy encoder unit;
a fingerprint collecting module including a display unit configured to display instructions for fingerprint capture;
a fingerprint scan unit configured to actuate a fingerprint scan;
a processing unit configured to process the fingerprint scan into a fingerprint template data set and store it in the temporary storage device;
the privacy encoder unit configured to combine and encrypt all data acquired from the temporary storage device into an encrypted data set by utilizing a private encryption key which is known only to the collecting apparatus; and
a purging module configured to purge from the temporary storage device all temporary data acquired by the photo collecting module and the fingerprint collecting module,
wherein the collecting apparatus is configured to encode, by hardware, raw biometric data acquired by the photo collecting module and the fingerprint collecting module, before delivery to the temporary storage device,
wherein the collecting apparatus is configured to delete the raw biometric data before delivery to the computing main board, and the collecting apparatus delivers resulting biometric data in an encoded and encrypted data set to the computing main board for permanent storage,
wherein the collecting apparatus is further configured to
require two immediate and simultaneous fingerprints, one from each of two persons;
record a response through an apparatus display and user-selectable response;
record biometric fingerprints from each of the two persons on each of two physical reader devices;
time-stamp a first fingerprint and a second fingerprint and electronically determine that the two fingerprints are recorded within a time period;
compute an electronic decision about the physical proximity of the two persons based upon at least a time-stamp of the first fingerprint and a time-stamp of the second fingerprint;
merge biometric signatures from the two persons into an electronic agreement;
generate a signed electronic agreement;
output the signed electronic agreement to the computing main board for permanent storage to memorialize the medical transaction declaration.

2. The collecting apparatus of claim 1, wherein the camera unit includes two cameras, one of the two cameras is for normal visible light and the other is for non-visible light.

3. The collecting apparatus of claim 2, wherein the two cameras are aligned to take a photo in the same direction.

4. The collecting apparatus of any one of claims 1 to 3, wherein the fingerprint scan unit includes two fingerprint readers, one of the two fingerprint readers is for one finger and the other is for multiple fingers.

5. The collecting apparatus of claim 4, wherein the two fingerprint readers are located proximate to each other.

6. A collecting method for recording a medical transaction declaration, comprising:
inputting patient information;
displaying countdown timer instructions for taking photos;
taking photos;
combining and hashing the photos into a combined data set and forward the combined data set to a temporary storage device on a privacy encoder unit;
displaying instructions for fingerprint capture;
actuating a fingerprint scan;
processing the fingerprint scan into a fingerprint template data set and storing it in the temporary storage device;
encoding, by hardware, raw biometric data acquired, before delivery to the temporary storage device;
combining and encrypting all data acquired from the temporary storage device into an encrypted data set by utilizing a private encryption key which is known only to a collecting apparatus;
purging from the temporary storage device all temporary data acquired,
deleting the raw biometric data before delivery to a computing main board, and
delivering resulting biometric data in an encoded and encrypted data set to the computing main board for permanent storage,
wherein the method further comprises
requiring two immediate and simultaneous fingerprints, one from each of two persons;
recording a response through an apparatus display and user-selectable response;
recording biometric fingerprints from each of the two persons on each of two physical reader devices;
time-stamping a first fingerprint and a second fingerprint and electronically determining that the two fingerprints are recorded within a time period;
computing an electronic decision about the physical proximity of the two persons based upon at least a time-stamp of the first fingerprint and a time-stamp of the second fingerprint;
merging biometric signatures from the two persons into an electronic agreement;
generating a signed electronic agreement;
outputting the signed electronic agreement to the computing main board for permanent storage to memorialize the medical transaction declaration.

7. The collecting method of claim 6, wherein the patient information includes last name, first name, and middle initial of the patient, identifying medical number of the patient, and date of birth of the patient; and the response includes an affirmative response and a negative response, and/or wherein the time period is 10 seconds.

8. The collecting method of claim 6, wherein the two persons include the patient and a witness.

9. The collecting apparatus of any one of claims 1 to 5, further comprising a casing module (1) in which the photo collecting module, the fingerprint collecting module, and the purging module are arranged, including filter vents, an exhaust vent screen (2), and an intake vent filter (3) configured to remove particulate matter.

10. The collecting apparatus of any one of claims 1 to 5 wherein the camera unit includes two cameras, a first of the two cameras being configured to detect humanly-visible light, and a second of the two cameras being configured to detect thermal, infrared or non-visible light spectrum.

11. The collecting method of claim 6, wherein:
at least a first of the acquired camera photographs represents detected humanly-visible light, and
at least a second of the acquired camera photographs represents detected thermal, infrared or non-visible light spectrum.

## Patentansprüche

1. Erfassungsvorrichtung zur Aufzeichnung einer medizinischen Transaktionserklärung, umfassend:
ein Eingabemodul, das zur Eingabe von Patienteninformationen konfiguriert ist;
eine Computer-Hauptplatine;
ein Fotoerfassungsmodul, das eine Anzeigeeinheit (4), die konfiguriert ist, um Countdown-Timer-Anweisungen zum Aufnehmen von Fotos anzuzeigen, eine Kameraeinheit (6), die konfiguriert ist, um Fotos aufzunehmen, eine Encodereinheit, die konfiguriert ist, um die Fotos zu einem kombinierten Datensatz zu kombinieren und zu hashen und den kombinierten Datensatz an ein temporäres Speichergerät auf einer Datenschutz-Encodereinheit weiterzuleiten, einschließt;
ein Fingerabdruck-Erfassungsmodul, das eine Anzeigeeinheit einschließt, die konfiguriert ist, um Anweisungen zur Fingerabdruckerfassung anzuzeigen;
eine Fingerabdruck-Scaneinheit, die konfiguriert ist, um einen Fingerabdruck-Scan auszuführen;
eine Verarbeitungseinheit, die konfiguriert ist, um den Fingerabdruck-Scan zu einem Fingerabdruck-Vorlagendatensatz zu verarbeiten und diesen im Zwischenspeichergerät zu speichern;
wobei die Datenschutz-Encoder-Einheit konfiguriert ist, um alle vom Zwischenspeichergerät erfassten Daten zu kombinieren und in einen verschlüsselten Datensatz zu verschlüsseln, indem sie einen privaten Verschlüsselungsschlüssel verwendet, der nur dem Erfassungsgerät bekannt ist; und
ein Löschmodul, das konfiguriert ist, um alle temporären Daten, die vom Fotoerfassungsmodul und vom Fingerabdruckerfassungsmodul erfasst wurden, aus dem temporären Speichergerät zu löschen,
wobei die Erfassungsvorrichtung konfiguriert ist, um durch Hardware rohe biometrische Daten, die vom Fotoerfassungsmodul und vom Fingerabdruckerfassungsmodul erfasst wurden, vor der Übermittlung an die temporäre Speichervorrichtung zu codieren,
wobei die Erfassungsvorrichtung konfiguriert ist, um die rohen biometrischen Daten vor der Übermittlung an die Computer-Hauptplatine zu löschen, und die Erfassungsvorrichtung die resultierenden biometrischen Daten in einem codierten und verschlüsselten Datensatz zur dauerhaften Speicherung an die Computer-Hauptplatine übermittelt,
wobei die Erfassungsvorrichtung ferner konfiguriert ist, um
zwei sofortige und gleichzeitige Fingerabdrücke zu verlangen, einen von jeder der beiden Personen;
eine Antwort über eine Geräteanzeige und eine vom Benutzer wählbare Antwort aufzuzeichnen;
biometrische Fingerabdrücke von jeder der beiden Personen auf jedem der beiden physischen Lesegeräte aufzuzeichnen;
einen Zeitstempel für einen ersten Fingerabdruck und einen zweiten Fingerabdruck zu erstellen und elektronisch zu bestimmen, dass die beiden Fingerabdrücke innerhalb eines Zeitraums aufgezeichnet werden;
eine elektronische Entscheidung über die physische Nähe der beiden Personen auf der Grundlage mindestens eines Zeitstempels des ersten Fingerabdrucks und eines Zeitstempels des zweiten Fingerabdrucks zu berechnen;
die biometrischen Signaturen der beiden Personen zu einer elektronischen Vereinbarung zusammenführen;
eine unterzeichnete elektronische Vereinbarung zu erstellen;
die unterzeichnete elektronische Vereinbarung an die Computer-Hauptplatine zur dauerhaften Speicherung auszugeben, um die medizinische Transaktionserklärung zu dokumentieren.

2. Erfassungsvorrichtung nach Anspruch 1, wobei die Kameraeinheit zwei Kameras einschließt, wobei eine der beiden Kameras für normales sichtbares Licht und die andere für nicht sichtbares Licht bestimmt ist.

3. Erfassungsvorrichtung nach Anspruch 2, wobei die beiden Kameras so ausgerichtet sind, dass sie ein Foto in derselben Richtung aufnehmen.

4. Erfassungsvorrichtung nach einem der Ansprüche 1 bis 3, wobei die Fingerabdruck-Scaneinheit zwei Fingerabdruck-Lesegeräte einschließt, wobei eines der beiden Fingerabdruck-Lesegeräte für einen Finger und das andere für mehrere Finger bestimmt ist.

5. Erfassungsvorrichtung nach Anspruch 4, wobei die beiden Fingerabdruck-Lesegeräte nahe beieinander angeordnet sind.

6. Erfassungsverfahren zum Aufzeichnen einer medizinischen Transaktionserklärung, umfassend:
Eingeben von Patienteninformationen;
Anzeigen von Countdown-Timer-Anweisungen zum Aufnehmen von Fotos;
Aufnehmen von Fotos;
Kombinieren und Hashen der Fotos zu einem kombinierten Datensatz und Weiterleiten des kombinierten Datensatzes an ein temporäres Speichergerät auf einer Datenschutz-Encoder-Einheit;
Anzeigen von Anweisungen zur Fingerabdruckerfassung;
Auslösen eines Fingerabdruck-Scans;
Verarbeiten des Fingerabdruck-Scans zu einem Fingerabdruck-Vorlagendatensatz und Speichern desselben in dem temporären Speichergerät;
Codieren der erfassten rohen biometrischen Daten durch Hardware vor der Übermittlung an das temporäre Speichergerät;
Kombinieren und Verschlüsseln aller von dem temporären Speichergerät erfassten Daten zu einem verschlüsselten Datensatz unter Verwendung eines privaten Verschlüsselungsschlüssels, der nur einer Erfassungsvorrichtung bekannt ist;
Löschen aller erfassten temporären Daten aus dem temporären Speichergerät,
Löschen der rohen biometrischen Daten vor der Übermittlung an eine Computer-Hauptplatine und
Übertragen der resultierenden biometrischen Daten in einem codierten und verschlüsselten Datensatz an die Computer-Hauptplatine zur dauerhaften Speicherung,
wobei das Verfahren ferner Folgendes umfasst:
Anfordern von zwei sofortigen und gleichzeitigen Fingerabdrücken, jeweils einen von zwei Personen;
Aufzeichnen einer Antwort über eine Vorrichtungsanzeige und eine vom Benutzer wählbare Antwort;
Aufzeichnen biometrischer Fingerabdrücke von jeder der beiden Personen auf jedem von zwei physischen Lesegeräten;
Erstellen eines Zeitstempels eines ersten Fingerabdrucks und eines zweiten Fingerabdrucks und elektronisches Bestimmen, dass die beiden Fingerabdrücke innerhalb eines Zeitraums aufgezeichnet werden;
Berechnen einer elektronischen Entscheidung über die physische Nähe der beiden Personen auf der Grundlage mindestens eines Zeitstempels des ersten Fingerabdrucks und eines Zeitstempels des zweiten Fingerabdrucks;
Zusammenführen der biometrischen Signaturen der beiden Personen zu einer elektronischen Vereinbarung;
Erstellen einer unterzeichneten elektronischen Vereinbarung;
Ausgeben der unterzeichneten elektronischen Vereinbarung an die Computer-Hauptplatine zur dauerhaften Speicherung, um die medizinische Transaktionserklärung zu dokumentieren.

7. Erfassungsverfahren nach Anspruch 6, wobei die Patienteninformationen den Nachnamen, den Vornamen und die Initiale des zweiten Vornamens des Patienten, die medizinische Identifikationsnummer des Patienten und das Geburtsdatum des Patienten einschließen; und die Antwort eine positive Antwort und eine negative Antwort einschließt und/oder wobei der Zeitraum 10 Sekunden beträgt.

8. Erfassungsverfahren nach Anspruch 6, wobei die beiden Personen den Patienten und einen Zeugen einschließen.

9. Erfassungsvorrichtung nach einem der Ansprüche 1 bis 5, ferner umfassend ein Gehäusemodul (1), in dem das Fotoerfassungsmodul, das Fingerabdruckerfassungsmodul und das Löschmodul angeordnet sind, einschließlich Filteröffnungen, eines Abluftöffnungsgitters (2) und eines Ansaugöffnungsfilters (3), die konfiguriert sind, um Partikel zu entfernen.

10. Erfassungsvorrichtung nach einem der Ansprüche 1 bis 5, wobei die Kameraeinheit zwei Kameras einschließt, wobei eine erste der beiden Kameras konfiguriert ist, um für den Menschen sichtbares Licht zu erfassen, und eine zweite der beiden Kameras konfiguriert ist, um thermisches, infrarotes oder nicht sichtbares Lichtspektrum zu erfassen.

11. Erfassungsverfahren nach Anspruch 6, wobei:
mindestens ein erstes der aufgenommenen Kamerabilder erkanntes, für den Menschen sichtbares Licht darstellt, und
mindestens ein zweites der aufgenommenen Kamerabilder erkanntes thermisches, infrarotes oder nicht sichtbares Lichtspektrum darstellt.

## Revendications

1. Appareil de collecte pour l'enregistrement d'une déclaration de transaction médicale, comprenant :
un module de saisie configuré pour la saisie d'informations sur un patient ;
une carte mère informatique ;
un module de collecte de photos comportant une unité d'affichage (4) configurée pour afficher des instructions de compte à rebours pour la prise de photos, une unité de caméra (6) configurée pour prendre des photos, une unité d'encodage configurée pour combiner et hacher les photos en un ensemble de données combinées et transmettre l'ensemble de données combinées à un dispositif de stockage temporaire sur une unité d'encodage confidentielle ;
un module de collecte d'empreintes digitales comportant une unité d'affichage configurée pour afficher des instructions pour la capture d'empreintes digitales ;
une unité de balayage d'empreintes digitales configurée pour activer un balayage d'empreintes digitales ;
une unité de traitement configurée pour traiter le balayage d'empreintes digitales en un ensemble de données de modèle d'empreintes digitales et le stocker dans le dispositif de stockage temporaire ;
l'unité d'encodage confidentielle étant configurée pour combiner et chiffrer toutes les données acquises à partir du dispositif de stockage temporaire en un ensemble de données chiffrées en utilisant une clé de chiffrement privée qui n'est connue que de l'appareil de collecte ; et
un module de purge configuré pour purger du dispositif de stockage temporaire toutes les données temporaires acquises par le module de collecte de photos et le module de collecte d'empreintes digitales,
dans lequel l'appareil de collecte est configuré pour encoder, par le biais de matériel, des données biométriques brutes acquises par le module de collecte de photos et le module de collecte d'empreintes digitales, avant leur envoi au dispositif de stockage temporaire,
dans lequel l'appareil de collecte est configuré pour supprimer les données biométriques brutes avant leur envoi à la carte mère informatique, et l'appareil de collecte envoie des données biométriques résultantes dans un ensemble de données encodées et chiffrées à la carte mère informatique pour un stockage permanent,
dans lequel l'appareil de collecte est en outre configuré pour
demander deux empreintes digitales immédiates et simultanées, une de chacune de deux personnes ;
enregistrer une réponse à travers un affichage d'appareil et une réponse sélectionnable par un utilisateur ;
enregistrer des empreintes digitales biométriques de chacune des deux personnes sur chacun des deux dispositifs de lecture physiques ;
horodater une première empreinte digitale et une seconde empreinte digitale et déterminer électroniquement que les deux empreintes digitales sont enregistrées dans un laps de temps ;
calculer une décision électronique concernant la proximité physique des deux personnes en fonction d'au moins un horodatage de la première empreinte digitale et d'un horodatage de la seconde empreinte digitale ;
fusionner des signatures biométriques des deux personnes en un accord électronique ;
générer un accord électronique signé ;
transférer l'accord électronique signé à la carte mère informatique pour un stockage permanent afin de mémoriser la déclaration de transaction médicale.

2. Appareil de collecte selon la revendication 1, dans lequel l'unité de caméra comporte deux caméras, l'une des deux caméras étant destinée à la lumière visible normale et l'autre à la lumière non visible.

3. Appareil de collecte selon la revendication 2, dans lequel les deux caméras sont alignées pour prendre une photo dans la même direction.

4. Appareil de collecte selon l'une quelconque des revendications 1 à 3, dans lequel l'unité de balayage d'empreintes digitales comporte deux lecteurs d'empreintes digitales, l'un des deux lecteurs d'empreintes digitales étant destiné à un doigt et l'autre à plusieurs doigts.

5. Appareil de collecte selon la revendication 4, dans lequel les deux lecteurs d'empreintes digitales sont situés à proximité l'un de l'autre.

6. Procédé de collecte pour l'enregistrement d'une déclaration de transaction médicale, comprenant :
la saisie d'informations sur un patient ;
l'affichage d'instructions de compte à rebours pour la prise de photos ;
la prise de photos ;
la combinaison et le hachage des photos en un ensemble de données combinées et la transmission de l'ensemble de données combinées vers un dispositif de stockage temporaire sur une unité d'encodage confidentielle ;
l'affichage d'instructions pour la capture d'empreintes digitales ;
l'activation d'un balayage d'empreintes digitales ;
le traitement du balayage d'empreintes digitales en un ensemble de données de modèle d'empreintes digitales et son stockage dans le dispositif de stockage temporaire ;
l'encodage, par le biais de matériel, de données biométriques brutes acquises, avant leur envoi au dispositif de stockage temporaire ;
la combinaison et le chiffrement de toutes les données acquises à partir du dispositif de stockage temporaire en un ensemble de données chiffrées en utilisant une clé de chiffrement privée qui n'est connue que d'un appareil de collecte ;
la purge du dispositif de stockage temporaire de toutes les données temporaires acquises,
la suppression des données biométriques brutes avant leur envoi à une carte mère informatique, et
l'envoi de données biométriques résultantes dans un ensemble de données encodées et chiffrées à la carte mère informatique pour un stockage permanent,
dans lequel le procédé comprend en outre
la demande de deux empreintes digitales immédiates et simultanées, une de chacune de deux personnes ;
l'enregistrement d'une réponse à travers un affichage d'appareil et d'une réponse sélectionnable par un utilisateur ;
l'enregistrement d'empreintes digitales biométriques de chacune des deux personnes sur chacun des deux dispositifs de lecture physiques ;
l'horodatage d'une première empreinte digitale et d'une seconde empreinte digitale et la détermination électroniquement du fait que les deux empreintes digitales sont enregistrées dans un laps de temps ;
le calcul d'une décision électronique concernant la proximité physique des deux personnes en fonction d'au moins un horodatage de la première empreinte digitale et d'un horodatage de la seconde empreinte digitale ;
la fusion de signatures biométriques des deux personnes en un accord électronique ;
la génération d'un accord électronique signé ;
le transfert de l'accord électronique signé à la carte mère informatique pour un stockage permanent afin de mémoriser la déclaration de transaction médicale.

7. Procédé de collecte selon la revendication 6, dans lequel les informations sur un patient comportent le nom, le prénom et l'initiale du deuxième prénom du patient, le numéro médical d'identification du patient et la date de naissance du patient ; et la réponse comporte une réponse affirmative et une réponse négative, et/ou dans lequel le laps de temps est de 10 secondes.

8. Procédé de collecte selon la revendication 6, dans lequel les deux personnes comportent le patient et un témoin.

9. Appareil de collecte selon l'une quelconque des revendications 1 à 5, comprenant en outre un module de boîtier (1) dans lequel sont agencés le module de collecte de photos, le module de collecte d'empreintes digitales et le module de purge, comportant des évents de filtre, un tamis d'évent d'évacuation (2) et un filtre d'évent d'admission (3) configurés pour éliminer des particules.

10. Appareil de collecte selon l'une quelconque des revendications 1 à 5, dans lequel l'unité de caméra comporte deux caméras, une première des deux caméras étant configurée pour détecter la lumière visible par l'homme, et une seconde des deux caméras étant configurée pour détecter le spectre de lumière thermique, infrarouge ou non visible.

11. Procédé de collecte selon la revendication 6, dans lequel :
au moins une première des photographies de caméra acquises représente la lumière visible par l'homme détectée, et
au moins une seconde des photographies de caméra acquises représente le spectre de lumière thermique, infrarouge ou non visible détecté.
